# EUROPEAN PATENT SPECIFICATION

(11) **EP 3 719 119 B1**
(45) Date of publication and mention of the grant of the patent: **13.11.2024**
(21) Application number: 19382247.5
(22) Date of filing: 03.04.2019
(51) Int. Cl.: C12N 5/0786, A61K 35/15, A61P 19/02, A61P 21/00, C12N 5/00

(54) **TISSUE-SPECIFIC GROWTH FACTORS, METHOD OF PRODUCTION AND USES THEREOF**
GEWEBESPEZIFISCHE WACHSTUMSFAKTOREN, VERFAHREN ZUR HERSTELLUNG UND VERWENDUNGEN DAVON
FACTEURS DE CROISSANCE SPÉCIFIQUES À DES TISSUS, LEUR PROCÉDÉ DE PRODUCTION ET LEURS UTILISATIONS

(43) Date of publication of application: 07.10.2020
(73) Proprietor: Peaches S.L., 28050 Madrid (ES)
(72) Inventor: Lapuente Fernández, Juan Pedro, 28050 Madrid (ES); de Gregorio Santos, Juan Carlos, 28050 Madrid (ES); Desportes Bielsa, Paula, 28050 Madrid (ES); Fernández Vicente, Pablo, 28050 Madrid (ES); Cayuela Calvo, María, 28050 Madrid (ES); Blázquez Martinez, Alejandro, 28050 Madrid (ES); Dos Anjos Vilaboa, Severiano, 28050 Madrid (ES); Cayuela Calvo, Maria, 28050 Madrid (ES); Iglesias Moreno, Carmen, 28050 Madrid (ES); Gomez Cepa, Gonzalo, 28050 Madrid (ES); Lapuente Fernández, Juan Pedro, 28050 Madrid (ES); Desporte Bielsa, Paula, 28050 Madrid (ES); Fernández Vicente, Pablo, 28050 Madrid (ES); Sanz Moreno, Jara, 28050 Madrid (ES)
(74) Representative: Clarke, Modet y Cía., S.L.

(56) References cited:
- WO-A1-2014/149301
- ZHU PING ET AL: "Expression of CD147 on monocytes/macrophages in rheumatoid arthritis: its potential role in monocyte accumulation and matrix metalloproteinase production", ARTHRITIS RESEARCH AND THERAPY, BIOMED CENTRAL, LONDON, GB, vol. 7, no. 5, 23 June 2005 (2005-06-23), pages R1023 - R1033, XP021011644, ISSN: 1478-6354
- FUNAYAMA H ET AL: "Human monocyte- endothelial cell interaction induces platelet-derived growth factor expression", CARDIOVASCULAR RESEARCH, OXFORD UNIVERSITY PRESS, GB, vol. 37, no. 1, 1 January 1998 (1998-01-01), pages 216 - 224, XP002118702, ISSN: 0008-6363, DOI: 10.1016/S0008-6363(97)00224-1
- LE ADRIAN D ET AL: "Current Clinical Recommendations for Use of Platelet-Rich Plasma", CURRENT REVIEWS IN MUSCULOSKELETAL MEDICINE, HUMANA PRESS, INC, US, vol. 11, no. 4, 23 October 2018 (2018-10-23), pages 624 - 634, XP036630108, ISSN: 1935-973X, [retrieved on 20181023], DOI: 10.1007/S12178-018-9527-7
- C. BORSELLI ET AL: "Regenerative Medicine Special Feature: Functional muscle regeneration with combined delivery of angiogenesis and myogenesis factors", PROCEEDINGS OF THE NATIONAL ACADEMY OF SCIENCES, vol. 107, no. 8, 23 February 2010 (2010-02-23), pages 3287 - 3292, XP055066525, ISSN: 0027-8424, DOI: 10.1073/pnas.0903875106

## Description

### FIELD OF THE INVENTION

The invention belongs to the field of human or animal medicine, in particular to the tissue regeneration. It relates to a method for obtaining the co-culture of tissue-specific cells and purified monocytes. The invention also relates to the composition obtained by this method and its therapeutic applications.

### BACKGROUND OF THE INVENTION

Growth factors (GF) play an important role in promoting cellular differentiation and cell division. They are substances active in stimulating the growth of cells and tissues and they can be produced by many different types of tissues. Examples of these substances include insulin-like growth factors (somatomedins), which stimulate growth by mediating the secretion of growth hormone from the pituitary gland; epidermal growth factor, which stimulates the growth of epithelial cells; platelet-derived growth factor, which stimulates the growth of muscle cells and connective tissue cells; and nerve growth factor, which stimulates the growth of neuronal cells.

GF can originate from platelets, plasma, the bone matrix, osteocytes and osteoblasts, fibroblasts, and from bone marrow. It has been shown that GF from blood samples have important biological properties, such as tissue regeneration and decrease of diseases (Civinini A et al., 2010. Clin Cases Miner Bone Metab 7(3):194).

GP from blood samples generally are used in form of platelet-rich plasma (PRP), i.e. of the portion of plasma that, after centrifugation, is rich in platelets. PRP comprises growth factors and cytokines such as platelet-derived growth factor, transforming growth factor beta, fibroblast growth factor, insulin-like growth factor 1, insulin-like growth factor 2, vascular endothelial growth factor, epidermal growth factor, Interleukin 8, keratinocyte growth factor and connective tissue growth factor. The efficacy of growth factors in healing injuries, and the concentrations of the GP found within PRP are the theoretical basis for the use of PRP in tissue repair. The platelets collected in PRP are activated by the addition of thrombin and calcium chloride.

For many years, the PRP comprising grow factors and cytokines have been used in the treatment of sport injuries. This have led to different conclusions regarding the efficacy and efficiency in the treatment of injuries in different tissues, since the results have shown high variability, usually is associated with the patient's age. It has been disclosed that the growing factors concentration in PRP significantly decreases in subjects over 25 years old in healthy population, especially in men, (Evanson JR *et al,* 2014. Mil Med 179(7): 799). Furthermore, any disease of a subject may additionally alter the concentration of GF in PRP, key in the regeneration-recuperation process of a sport injury in soft or osteocartilaginous tissue.

Funayama investigated whether the synthesis of platelet-derived growth factor (PDGF) was induced by the direct cell-to-cell interaction between human monocytes and umbilical vein endothelial cells (ECs), concluding that the interaction between these cells induces PDGF synthesis, suggesting an important role in the pathogenesis of atherosclerosis (Funayama H et al., 1998. Cardiovascular Research 37; 216-24).

Zhu studied the effect of monocytes and macrophages in rheumatoid arthritis pathogenesis, activating fibroblasts through CD147 expression on CD14+ monocytes. The authors studied the role of CD147 in metalloprotease production and cell invasion through the co-culture of human monocytes and human fibriblasts.

All medical products in the prior art comprising GF and directed to tissue regeneration are autologous, this is, the same individual is the cell donor and the recipient. One of the more relevant features of these compositions is that they must be applied into the patient immediately after their preparation, in order to avoid losing the biological properties of the GF. During the storage of these medical products changes in the GF's properties, such as lowering pH, increase of the potassium level in plasma and changes in their metabolism, which affect the biological effect of the GF (Martinez MC et al. Transfusion medical applications and practice. 2012. First edition. Cortés A. Vol I, chapter 4, p. 71).

Moreover, since blood compounds cannot survive at room temperature for long periods of time, it is recommended to apply compositions comprising thereof immediately. The recommended period between the patient's blood extraction and the PRP administration should be of no more than 45 minutes (Moreno R et al., 2015. Farm Hosp 39(3):130). A plasma supernatant must be used within eight hours from its preparation in order to avoid degradation or denaturalization of the growing factors and also avoid bacterial contamination. All the process is performed in sterile and aseptic conditions, since the composition comprising the PRP will be introduced in the subject's body.

In order to administering the GF to a patient, recent medical applications of autologous blood growth factors require infiltration or continuous administration of the product with a short interval between doses, in order the product may be effective. For these applications, the preparation of the composition comprising autologous blood compounds, requires continuous blood extraction from the patient, since fresh compositions must be prepared each time the composition is needed. This affects negatively to the patient's life quality and make this procedure unsuitable in long-term treatments for chronic or degenerative conditions. Therefore, it would be an advantage to have a composition comprising GF able to be stored while its components remain stable and biologically active over time. This would allow the storage of the product, without having to apply to the patient immediately.

In the sports field, the patient injured requires the administration of a tissue-repairing composition as soon as possible, preferably immediately after he/she has suffered the injury, in order the patient's recovery may start as soon as possible. This is especially important in high-level athletes. A composition stored would allow to treat an injury immediately after it has occurred, which would benefit the recovery prognosis and the patient's quality of life, since no additional blood extractions would be needed.

Due to the compulsory need of frequent administration of autologous PRP compositions to a patient, there are multiple chronic or degenerative pathologies which could be potentially treated with these compositions, but they have not been treated due to the continuous requirements from the patient, negatively affecting his/her quality of life. Examples of these diseases are eye pathologies, pathologies of the central nervous system, degenerative joint pathologies, rheumatic pathologies, endocrine pathologies, sports injuries and any other disease or pathology which would require the repeated administration of a blood derived composition containing GF.

Moreover, PRP compositions deriving from platelets show an additional drawback: the growing factors, cytokines and chemokines obtained from these cells show a very high variability, both quantitative and qualitative, which make difficult to obtain reproducible batches of composition. The varying GF concentration may result in different biologic effects. Thus, the GF levels in each composition should be analyzed for reliable interpretation of the biologic functions and standardized application of PRP (Soon et al., 2011. Korean J Lab Med 31: 212). In fact, it has been shown that other factors such as aging, diet, lifestyle, stress, illnesses, etc. negatively affect the content thereof (Bai MY et al., 2018. Scientific Reports 8:10642).

Another interesting aspect to consider in the regeneration or repair of a tissue through the use of compositions comprising growth factors, is their pleiotropism. Although it is possible to assign to each type of GF a main property, its effect on each tissue is conditioned by the presence of other components in the composition, such as other growth factors or cytokines, which may produce an effect opposite to the desire effect for an specific growth factor. The pleiotropism is due to the fact that the cellular responses to the different cytokines and growth factors are activated when said compounds activate their receptors in the target cells. Each cytokine or each factor is typically recognized by only 1 or 2 different types of receptors and the interaction is highly specific, although it can be modulated by different co-adjuvants or antagonists of other cytokines and/or factors.

Consequently, there is therefore a need for a blood-derived composition suitable to be used tissue regeneration comprising growth factors and/or cytokines, this composition able to be stored for long periods of time, while the growth factors and/or cytokines maintain their function and biological activity. The composition should be suitable for allogenic administration and be efficient in tissue regeneration.

The present invention provides a solution for this problem disclosing a process for obtaining a composition comprising growth factors and/or cytokines, the composition obtained by said method being able to be stored and biologically active for at least one year. This composition may be applied immediately after its obtention of in any other moment to a subject needed thereof.

### DESCRIPTION OF THE INVENTION

### Brief description of the invention

The present invention is set out in the appended set of claims.

In a first aspect, it is disclosed a method for obtaining a composition comprising the following steps:
a) purification of monocytes from a sample in sterile conditions;
b) culturing the purified monocytes in serum free medium with tissue-specific cells;
c) collection the supernatant comprising growth factors and/or cytokines produced by the culture of step b); and
d) cryopreservation of the supernatant collected in step c) at a temperature between -35 and -165 °C.

wherein the tissue-specific cells are selected from the group consisting of myocytes, skeletal muscle cells, muscle satellite cells, tenocytes, Schwann cells, chondrocytes, synoviocytes, lymphocytes, hair follicle cells, myocardia cells, mesenchymal stem cells or combinations thereof;
wherein the composition is for tissue regeneration of musculoskeletal injuries.

In an embodiment, the method additionally comprises the step e) of reconstitution of the growth factors cryopreserved by maintaining the composition between 25 and 30 °C between for 10 and 60 minutes.

In another embodiment, the invention discloses the composition for tissue regeneration obtained obtained by the method disclosed above.

In another embodiment, the invention discloses a pharmaceutical composition comprising the composition obtained by the method of the invention.

In another embodiment, the invention discloses a composition obtainable by a method consisting of the following steps:
a) purification of monocytes from a sample;
b) culturing the purified monocytes in serum free medium with tissue specific cells selected from the group consisting of human chondrocytes, human synoviocytes, human Schwann cells, human skeletal muscle cells, human muscle satellite cells, human dermal fibroblasts, Human tenocytes, human mesenchymal stem cells; and; and
c) collection of the supernatant comprising growth factors and/or cytokines produced in the culture of step b);
for use in a method of treatment a musculoskeletal disease selected from tendinosis, muscle fibrillary break, tendinitis, bursitis, synovitis, arthrosis, cartilage break or chondropathies
wherein the composition is administered in a tissue comprising cells of the same type as the tissue-specific cells of step b).

In another embodiment the invention discloses a composition obtainable by a method comprising the following steps:
a) purification of monocytes from a sample in sterile conditions;
b) culturing the purified monocytes in serum free medium with tissue-specific cells selected from the group consisting of human chondrocytes, human synoviocytes, human Schwann cells, human skeletal muscle cells, human muscle satellite cells, human dermal fibroblasts, Human tenocytes, human mesenchymal stem cells; and
c) collection of the supernatant comprising growth factors and/or cytokines produced in the culture of step b);
d) cryopreservation of the supernatant collected in step c) at a temperature between -35 and -165 °C;
e) reconstitution of the supernatant of step d) by maintaining it between 25 and 30 °C,

for use in a method of treatment a disease selected from tendinosis, muscle fibrillary break, tendinitis, bursitis, synovitis, arthrosis, cartilage break, or a chondropathy;
wherein step e) is performed for 10 to 60 minutes prior to its use, and
wherein the composition is administered in a tissue comprising cells of the same type as the tissue-specific cells of step b).

In another embodiment, the composition for use is administered to a subject different from the subject donor of the monocytes or the growth factor stimulating cells.

In another embodiment, the composition for use is cryopreserved after step c) and reconstituted by maintaining the composition between 25 and 30 °C between for 10 and 60 minutes prior to its use.

In another embodiment, the composition for use is administered in a tissue comprising cells of the same type as the growth factor stimulating cells of step b).

### Description of the figures

The following figures illustrate the present invention.
**Figure 1****.** A: Fastimmune Cytokine Assay with PRP. B: Fastimmune Cytokine Assay with a composition of the invention. The cells are detected with CD33 antibody. R1 corresponds to lymphocytes and R2 corresponds to monocytes.
**Figure 2****.** Concentration (ng/mL) of platelet-derived growth factor AA (PdGF AA) in the composition of the invention (PRS, black column) and platelet-rich plasma (PRP, grey column).
**Figure 3****.** Concentration (ng/mL) of platelet-derived growth factor AB (PdGF AB) in the composition of the invention (PRS, black column) and platelet-rich plasma (PRP, grey column).
**Figure 4****.** Concentration (ng/mL) of platelet-derived growth factor BB (PdGF BB) in the composition of the invention (PRS, black column) and platelet-rich plasma (PRP, grey column).
**Figure 5****.** Concentration (ng/mL) of transforming growth factor beta (TGF beta) in the composition of the invention (PRS, black column) and platelet-rich plasma (PRP, grey column).
**Figure 6****.** Concentration (ng/mL) of vascular endothelial growth factor (VEGF) in the composition of the invention (PRS, black column) and platelet-rich plasma (PRP, grey column).
**Figure 7****.** Concentration (ng/mL) of epidermal growth factor (EGF) in the composition of the invention (PRS, black column) and platelet-rich plasma (PRP, grey column).
**Figure 8****.** Concentration (pg/mL) of insulin-like growth factor 1 (IGF 1) in the composition of the invention (PRS, black column) and platelet-rich plasma (PRP, grey column).
**Figure 9****.** Concentration (pg/mL) of interleukin 1 beta (IL 1 beta) in the composition of the invention (PRS, black column) and platelet-rich plasma (PRP, grey column).
**Figure 10****.** Concentration (pg/mL) of interleukin 6 (IL 6) in the composition of the invention (PRS, black column) and platelet-rich plasma (PRP, grey column).
**Figure 11****.** Concentration (pg/mL) of interleukin 10 (IL 10) in the composition of the invention (PRS, black column) and platelet-rich plasma (PRP, grey column).
**Figure 12** Concentration (pg/mL) of the tumor necrosis factor alpha (TNF alpha) in the composition of the invention (PRS, black column) and platelet-rich plasma (PRP, grey column).
**Figure 13****.** Evolution of the percentage of patients suffering from patellar tendinitis of phase II (II, black) and phase III (III, white) from day 0 (control) until day 27 after treatment.
**Figure 14****.** Evolution of the percentage of patients suffering from Achilles tendinitis of phase II (II, black) and phase III (III, white) from day 0 (control) until day 30 after treatment.
**Figure 15****.** Evolution of the percentage of patients suffering from muscle ruptures of phase II (II, black) and phase III (III, white) from day 0 (control) until day 42 after treatment.
**Figure 16****.** Echography of a subject with a muscle rupture done on the day previous to the treatment (-1), the day 4 after the treatment (+4) and the day 8 after the treatment (+8).
**Figure 17****.** Evolution of the percentage of patients suffering from gonarthrosis of phase II (II, black) and phase III (III, white) from day 0 (control) until day 56 after treatment.
**Figure 18****.** Evolution of the percentage of patients suffering from chondromalacia patellae of phase II (II, black) and phase III (III, white) from day 0 (control) until day 56 after treatment.
**Figure 19****.** Evolution of the percentage of patients suffering from anterior cruciate ligament rupture of phase II (II, black) and phase III (III, white) from day 0 (control) until month 5 after treatment.
**Figure 20****.** Evolution of the percentage of patients suffering from dynamic osteopathy of the pubis of phase II (II, black), phase III (III, white) and phase IV (IV, grey) from day 0 (control) until month 4 after treatment.
**Figure 21****.** Evolution of the percentage of patients suffering from astragalus fibular ligament sprain of phase II (II, black) and phase III (III, white) from day 0 (control) until day 24 after treatment.

### Detailed description of the invention

In an embodiment, the present invention discloses a method for obtaining a composition comprising the following steps:
a) purification monocytes from a sample in sterile conditions;
b) culturing the purified monocytes in serum free medium with tissue-specific cells;
c) collection the supernatant comprising growth factors and/or cytokines produced by the culture of step b); and
d) cryopreservation of the supernatant collected in step c) at a temperature between -35 and -165 °C.

wherein the tissue-specific cells are selected from the group consisting of myocytes, skeletal muscle cells, muscle satellite cells, tenocytes, Schwann cells, chondrocytes, synoviocytes, lymphocytes, hair follicle cells, myocardia cells, mesenchymal stem cells or combinations thereof;
wherein the composition is for tissue regeneration of musculoskeletal injuries.

In a preferred embodiment, after having been stored, the method additionally comprises the step of
e) reconstitution of the growth factors and/or cytokines cryopreserved approximately between 25 and 30 °C for approximately between 10 and 60 minutes.

The method of the invention is directed to the obtention of a composition which will be used in tissue regeneration.

For purposes of the present invention, the expressions "regenerative medicine", "tissue repair", "injuries in a tissue", "tissue recuperation", "tissue regeneration" or "tissue healing" should be considered synonymous of any process which implies the healing of a tissue comprised of mammal cells previously injured in any way. Preferably, the process implies the regeneration of the wounded tissue.

Prior to the purification of the monocytes in step a), the sample of monocytes may be obtained by either by apheresis, from whole blood by gradient centrifugation with a ficoll medium, or by any other technique known in the art. Apheresis is an extracorporeal medical technology in which the blood of a person is passed through an apparatus that separates out one particular constituent and returns the remainder to the circulation. Blood is filtered to remove the stem cells. Centrifugation by ficoll medium (Ficoll-Plaque) is a technique which separates blood to its components. The medium is normally placed at the bottom of a conical tube, and blood is then slowly layered above the medium. After being centrifuged, the following layers are visible in the conical tube, from top to bottom: plasma and other constituents, a layer of mono-nuclear cells called buffy coat (PBMC/MNC).

The purification of the monocytes is performed by placing them in culture under hypoxic conditions of total sterility. Since the composition obtained by the process of the invention will be applied in a subject, it is necessary that all the steps are performed under sterile conditions. In the body, oxygen concentrations range from 1 to 12%, rather than the 21 % in the atmosphere. Cells cultured in low oxygen, or hypoxia, grow faster, live longer and show lower stress. The oxygen concentration in hypoxic conditions is 3 - 5 %. Monocytes may be selected, for example, by adherence after a 24-hour incubation culture or by a second centrifugation in a density gradient.

In step b), the monocytes purified in step a) are cultured in a serum free medium with a tissue-specific cell (also known as co-culture). For purposes of this invention, tissue-specific cells and growth factor stimulating cells refers to the same concept, this is, one or more cells specific from a tissue. The co-culture of the monocytes and the tissue-specific cells stimulates growth factor and/or cytokine production during this culture. It is very important to avoid any ingredient which may induce monocyte differentiation, such as bovine fetal serum, phenol red, antibiotics or antifungals among others. The growth factors and/or cytokines produced in the supernatant of the culture are the essential elements of the composition of the invention. In a preferred embodiment, the culture medium further contains macrophage colony-stimulating factor (M-CSF). The origin of the monocytes is a blood sample from the same or a different subject donor of the growth factor stimulating cells. In a preferred embodiment, the origin of the monocytes is a blood sample from the same subject donor of the growth factor stimulating cells.

In a preferred embodiment, the tissue-specific cells or growth factor stimulating cells are cells selected from: myocytes, skeletal muscle cells, muscle satellite cells, tenocytes, Schwann cells, dermal fibroblasts, chondrocytes, synoviocytes, lymphocytes, hair follicle cells, myocardia cells, mesenchymal stem cells or combinations thereof. In a preferred embodiment, the cells are from human origin. The cells may be obtained from a cell collection, or from a primary cell culture from a tissue of a subject (donor). The cells of the tissue may be healthy or diseased cells.

The culture of the monocytes and the tissue-specific cells may further comprise adult mesenchymal cells, being from the same or different origin (cell collection or primary cell culture from the tissue of a donor) than the tissue-specific cells (autologous or allogenic adult mesenchymal cells). In a preferred embodiment, the purified monocytes are cultured in step b) with a primary culture of cells of a tissue which can be the same tissue in which the composition obtained will be applied in a subject. During the culture, which is also performed in sterility with hypoxia, the monocytes produce the growth factors and/or cytokines and excrete them to the supernatant, from which they are collected. Depending on the tissue-specific cells added to the culture, it is possible to obtain tissue-specific sets of growth factors and/or cytokines. The cells specific of a given tissue induce the production of growth factors and/or cytokines different from a different tissue. This allow to prepare compositions having different types of growth factors and/or cytokines depending on the tissue-specific cells in step b).

In a preferred embodiment the co-culture in step b) is performed between 30 and 40°C, preferably at 37 °C.

The collection of the growth factors and/or cytokines in step c) may be done by any method known by a person skilled in the art. These components are excreted in the culture media during the co-culture and are specific of the tissue from which the tissue-specific cells cultured with the monocytes come from. The process may further comprise the nanoencapsulation of the growth factors collected. Nanoencapsulation of therapeutic agents increases their efficacy, specificity and targeting ability. Nanocarriers protect their payload from premature degradation in the biological environment, enhance bioavailability, and prolong presence in blood and cellular uptake. In a preferred embodiment, the growth factors are encapsulated by, microfluidic technology. In a preferred embodiment, the collection of the composition from the culture in step c) is carried out at a temperature between 10 and 20 °C, more preferably at 15 °C. These low temperatures in step c) avoid or minimize the loss of volatile constituents present in the plasma or in the culture supernatant and also allow maintaining a very low risk of microbial contamination in the composition.

The cryopreservation of the product in step d) is carried out by freezing the supernatant obtained in step c) at a temperature between -35 and -165 °C. In a preferred embodiment, the composition is frozen at -35 °C and then cryopreserved at a temperature between -35 °C to -86 °C. In an even more preferred embodiment, the composition is frozen at -35 °C and then submitted to a temperature ramp until -86 °C. In a most preferred embodiment, the composition is first frozen and cryopreserved at 35 °C for 24 hours and then, gradually submitted to lower temperatures until -86 °C. Preferably, the composition is stored between -80 and -86 °C until its use. Storage from -40 °C has been experimentally proved to ensure stability and biological activity of the GF and/or cytokines present in the composition and their activity *in vivo* restoring an injured tissue as well. The composition cryopreserved may be maintained at least 1 year between -40 and -165 °C. Preferably at least 3 years. In a preferred embodiment, the supernatant is dosed in single doses.

The composition of the invention, like any other tissue regeneration composition, should be applied at room temperature (25 - 30 °C). This temperature is essential, since the purpose of the composition is for use as a treatment in regenerative medicine, which are performed always at these temperatures, since the treatment is applied directly in the injured tissue of a subject. For this reason, the reconstitution of the composition in step e) should be carried out by maintaining the composition at room temperature (between 25 and 30 °C) for between 10 minutes and 1 hour. In a preferred embodiment, the reconstitution of the composition performed by maintaining it between 25 and 30 °C for between 20 and 40 minutes. The defrost of the composition during the reconstitution step e) causes the destruction of any fraction of the complement and any immunoglobulin present in the sample. The presence of immunoglobulins in the composition may produce immunological cross-reactions and autoimmune reactions in the subject receptor of the composition. Therefore, absence of immunoglobulins in the defrosted composition allows the product obtained by this process to be applied to a receptor subject suffering from an autoimmune disease. Consequently, the composition of the invention could be used in the subject donor of the tissue-specific cells (autologous) or in a different subject (allogenic), without risk of immune reaction in the recipient.

The composition reconstituted after step e) has an analogous concentration of growth factors and/or cytokines with the same biological activity, in relation to the composition obtained after step c). Therefore, the composition obtained under the method disclosed herein is stable over the time after cryopreservation and subsequent reconstitution. This stability and effectivity of the composition after cryopreservation allows storage of the composition and avoid any subsequent blood extraction to the donor, since doses of the composition obtained by the process described herein may be applied subsequently according to a patient's needs (the same subject as the donor or any other recipient).

It has been also experimentally proved that the effects on cell proliferation, cell migration, chemotaxis and auto and paracrine synthesis of growth factors induced by the reconstituted composition are identical to those of the composition before cryopreservation. Therefore, the cryopreservation and reconstitution of the composition have no effect in the receptor tissue.

In a preferred embodiment, the composition obtained by the method of the invention contains platelets, red cells or leukocytes in an amount of less than 1 % of the total amount of components in the sample. More preferably less than 0.5 % and even more preferably, the composition contains less than 0.001 % of platelets, red cells or leukocytes of the total amount of components in the sample.

In another embodiment, the disclosure is directed to a composition for tissue regeneration obtained by a method consisting of the following steps:
a) purification of monocytes from a sample in sterile conditions;
b) culturing the purified monocytes in serum free medium with tissue-specific cells;
c) optionally, adding a macrophage colony-stimulating factor;
d) collection the supernatant comprising growth factors and/or cytokines produced by the culture of step b);
e) cryopreservation of the supernatant collected in step c) at a temperature between -35 and -165 °C,
wherein the tissue-specific cells are selected from the group consisting of myocytes, skeletal muscle cells, muscle satellite cells, tenocytes, Schwann cells, chondrocytes, synoviocytes, lymphocytes, hair follicle cells, myocardia cells, mesenchymal stem cells or combinations thereof.

In a preferred embodiment, the composition comprises platelets, red cells or leukocytes in an amount of less than 0.001% of the total amount of components in the sample.

Another aspect of the invention refers to a pharmaceutical composition comprising the composition obtained by the method disclosed above and a pharmaceutically acceptable carrier. As would be appreciated by one of skill in this art, the carriers may be chosen based on the route of administration as described below, the location of the target issue, the drug being delivered, the time course of delivery of the drug, etc. In an embodiment, the pharmaceutically acceptable carrier is selected so as not to unduly affect the biological activity of the composition of the invention. They may include emulsifiers, salts, pharmacologically acceptable buffers, binders, glidants, lubricants, disintegrants, sweeteners, pigments, co-solvents, surfactants, oils, humectants, emollients, preservatives, stabilizers, antioxidants or combinations thereof.

The pharmaceutical composition can be administered to a subject by any means known in the art, preferably directed to the injured tissue. The term "subject" as used herein, refers to humans as well as non-humans. For instance, the non-humans may be mammals (*e.g*., a rodent, a mouse, a rat, a rabbit, a monkey, a dog, a cat, a primate, or a pig).

In an embodiment, the composition or pharmaceutical composition disclosed herein may be storage in an injectable sterile vial. The composition or pharmaceutical composition may be administered directly to the injured tissue. In a more preferred embodiment, the composition is administered, for example, via intra-articular, subdermal, subcutaneous, intravenous or intratendinous.

In another embodiment, the invention discloses a composition obtainable by a method consisting of the following steps:
a) purification monocytes from a sample;
b) culturing the purified monocytes in serum free medium with tissue-specific cells selected from the group consisting of human chondrocytes, human synoviocytes, human Schwann cells, human skeletal muscle cells, human muscle satellite cells, human dermal fibroblasts, Human tenocytes, human mesenchymal stem cells; and
c) collection the supernatant comprising growth factors and/or cytokines produced in the culture of step b);

for use in a method of treatment a musculoskeletal disease selected from tendinosis, muscle fibrillary break, tendinitis, bursitis, synovitis, arthrosis, cartilage break or chondropathies
wherein the composition is administered in a tissue comprising cells of the same type as the tissue-specific cells of step b).

In a preferred embodiment, the composition for use is administered to a subject different from the subject donor of the monocytes or the tissue-specific cells.

In another embodiment, the invention discloses a composition obtainable by a method consisting of the following steps:
a) purification of monocytes from a sample in sterile conditions;
b) culturing the purified monocytes in serum free medium with tissue-specific cells selected from the group consisting of human chondrocytes, human synoviocytes, human Schwann cells, human skeletal muscle cells, human muscle satellite cells, human dermal fibroblasts, Human tenocytes, human mesenchymal stem cells;
c) collection the supernatant comprising growth factors and/or cytokines produced in the culture of step b);
d) cryopreservation of the supernatant collected in step c) at a temperature between -35 and -165 °C; and
e) reconstitution of the supernatant of step d) by maintaining it between 25 and 30 °C,

for use in a method of treatment a disease selected from tendinosis, muscle fibrillary break, tendinitis, bursitis, synovitis, arthrosis, cartilage break or chondropathies
wherein step e) is performed for 10 to 60 minutes prior to its use, and
wherein the composition is administered in a tissue comprising cells of the same type as the tissue-specific cells of step b).

The steps of the method for obtaining the composition for use as indicated above should be interpreted as previously described for other embodiments in the present document.

The composition, or pharmaceutical composition, is for use in the treatment of a disease in needed of regenerative medicine, tissue repair, tissue regeneration or tissue healing. The disease may be any disease which requires tissue healing after having suffered any kind of traumatic event. The term "repair", when used in the context of the healing of damaged tissue, is defined as the restoration of tissue architecture and function after an injury. Certain tissues of the body are more capable of cellular proliferation (and hence regeneration) than others. Regenerative medicine is defined as the treatment of a disease which require regeneration of biological tissues.

The composition described herein is for use in the treatment of tissues which have been injured and require tissue regeneration. In a preferred embodiment, the growth factors and/or cytokines present in the composition are tissue-specific. This means that they have been produced in a culture comprising monocytes and, as growth factor stimulating cells, cells of the same type of the cells which form part of the tissue to be recovered/treated/regenerated. This allows specific treatment to a tissue with specific growth factors and/or cytokines produced by cells of the same tissue. It has been experimentally demonstrated that the tissue-specific compositions - have improved biological properties, producing better results *in vitro* and *in vivo* in tissue regeneration.

In a more preferred embodiment, the composition is for use in the treatment of a disease which belongs to the field of sport medicine, traumatology, repair or rehab. In a more preferred embodiment, the composition is for use in tendinosis, muscle fibrillary break, tendinitis, bursitis, synovitis, arthrosis, cartilage break, or a chondropathy.

In a more preferred embodiment, the composition, or a pharmaceutical composition for use in regenerative medicine is directed to the repair of a tissue selected from the group comprising cartilaginous tissue, bone tissue, synovial tissue, fatty tissue, tendon tissue, ligamentous tissue, muscle tissue, epithelial tissue or endothelial tissue.

In a specific embodiment, the composition for use in the repair of cartilaginous tissue has been obtaining by using as growth factor stimulating cells, chondrocytes and/or chondroblasts.

In another specific embodiment, the composition for use in the repair of bone tissue has been obtaining by using as growth factor stimulating cells, osteoblasts and/or osteocytes and/or osteoclasts.

In another specific embodiment, the composition for use in the repair of synovial tissue has been obtaining by using as growth factor stimulating cells, fibroblasts synovial cells or type B cells and/or macrophage-like synovial cells.

In another specific embodiment, the composition for use in the repair of tendon tissue has been obtaining by using as growth factor stimulating cells, tenocytes (fibrocytes) and/or tenoblasts (fibroblasts).

In another specific embodiment, the composition for use in the repair of ligamentous tissue has been obtaining by using as growth factor stimulating cells, periodontal ligament stem cells and/or fibrocytes.

In another specific embodiment, the composition for use in the repair of muscle tissue has been obtaining by using as growth factor stimulating cells, myocytes.

In another specific embodiment, the composition for use in the repair of epithelial tissue has been obtaining by using as growth factor stimulating cells, epithelial cells and/or fibroblasts.

In another specific embodiment, the composition for use in the repair of endothelial tissue has been obtaining by using as growth factor stimulating cells, endothelial cells.

In a more preferred embodiment, the composition for use described in the previous embodiments, is directed to the treatment of patellar tendinosis, partial muscular ruptures, Achilles tendonitis, gonarthrosis, patellar chondropathy, anterior cruciate ligament tear, dynamic pubic osteopathy or sprained astragalus ligament.

In another embodiment, the invention disclosed a method for treatment a disease which requires tissue regeneration, comprising administering an effective pharmaceutical amount of the pharmaceutical composition described above. In a preferred embodiment, the disease is selected from the group comprising tendinosis, muscle fibrillary break, tendinitis, bursitis, synovitis, chronic neuropathy pain, ulcers, dermal regeneration, arthrosis, cartilage break, paralysis, neurological injuries or chondropathies or any other disease which require regenerative medicine.

In a preferred embodiment, the composition for use according to the previous embodiments is administered to a subject different from the subject donor of the monocytes or the growth factor stimulating cells, this is, the composition is for allogenic use.

In an embodiment, the composition for use obtainable by the method indicated above is applied in the target tissue after completing the method of its obtention. Alternatively, in a preferred embodiment, the composition for use is cryopreserved after step c) and reconstituted by prior its use in the treatment of the disease. In a more preferred embodiment, the composition is reconstituted by maintaining the composition between 25 and 30 °C between for 10 and 60 minutes.

In preferred embodiment, the composition for use is administered in a tissue comprising cells of the same type as the growth factor stimulating cells of step b).

The composition obtained by the method disclosed herein has proven to be effective in healing damaged tissues, reducing the time of recovering of the subject comprising the tissue damage. The main reason behind the success of the composition is the present of specific growth factors for each specific tissue and the maintenance of their biological function despite having been cryopreserved.

In the present disclosure, the technical and scientific terms used in the descriptions herein will have the meanings commonly understood by one of ordinary skill in the art, unless specifically defined otherwise.

### EXAMPLES

The examples described below have as an object to illustrate this invention, but without limiting the scope thereof.

### Example 1: Preparation of the composition

Bags of monocytic apheresis were centrifuged in a density gradient with Ficoll 30 minutes at 400 xg. The intermediate band of peripheral blood mononuclear cells (PBMC) was collected. The fraction was washed four times with physiological serum. The cells were resuspended in serum. The cells were cultured in T175 plates for 24 hours at 37 °C and the monocytes were selected by adherence. The cells were centrifuged in a Percoll density gradient.

The monocytes were extracted from the gradient fraction and cultured in GMP medium (GIBCO^{®} GTS^{™} AIM V^{®} SFM, Thermofisher) without phenol red and without antibiotics. 350 µL of growth factor supplement was added.

Primary cultures of human chondrocytes, human synoviocytes, human Schwann cells, human skeletal muscle cells, human muscle satellite cells, human dermal fibroblasts were provided by Innoprot. Human tenocytes were obtained from Zenbio and the human mesenchymal stem cells come from primary cultures obtained from samples of abdominal fat tissue.

The cells were cultured at 37 °C in poly-lysine pre-treated culture flasks in culture media as described below:
- Human tenocytes: 100 mL DMEM, 240 µL heparin, 5 mL lysed platelets, 1 mL antibiotics, 1 mL glutamine, 0.5 mL amphotericin B.
- Mesenchymal stem cells: 100 mL alpha-MEM, 240 µL heparin, 5 mL lysed platelets, 1 mL glutamine, 0.5 mL amphotericin B.
- Human skeletal muscle cells v human muscle satellite cells: 100 mL DMEM, 240 µL heparin, 5 mL lysed platelets, 1 mL skeletal muscle cell growth supplement, 1 mL antibiotics.
- Human chondrocytes: 100 mL DMEM, 240 µL heparin, 5 mL lysed platelets, 1 mL CGS (Chondrocyte Growth Supplement), 1 mL antibiotics.
- Human Schwann cells: 100 mL DMEM, 240 µL heparin, 5 mL lysed platelets, 1 mL SCGS (Schwann Cells Growth Supplement), 1 mL antibiotics.
- Human dermal fibroblasts: 100 mL DMEM, 240 µL heparin, 2 mL lysed platelets, 1 mL FGS (Fibroblast Growth Supplement), 1 mL antibiotics.
- Human synoviocytes: 100 mL DMEM, 240 µL heparin, 2 mL lysed platelets, 1 mL SGS (Synoviocyte Growth Supplement), 1 mL antibiotics.

### Example 2: Analysis of the composition

The components of a 24-hour co-culture of monocytes obtained after a co-culture were analyzed and compared with the components of a control composition of PRP after platelet degranulation.

When the cells have been previously frozen, the defrost was carried out by seeding the cells in 1T75 plates with PLL and adding 20 mL of culture media, except for human tenocytes and mesenchymal stem cells, which were centrifugated at 400 xg for 7 minutes prior to adding the cells to the culture media.

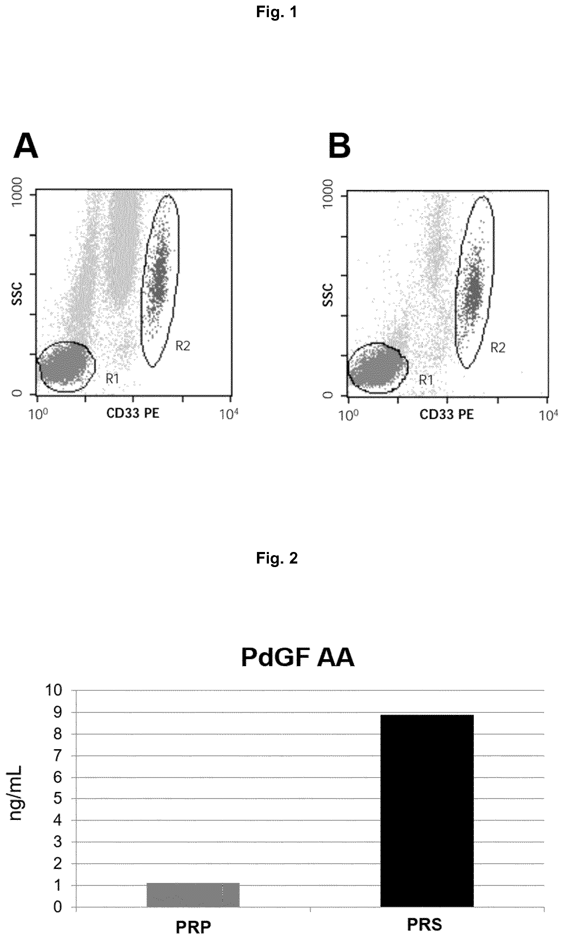

The components were detected in a flow cytometry apparatus CUBE 6 with the Fastimmune Cytokine Assay kit. Figure 1 shows that the general molecular content of the composition (A) is similar to the PRP control (B). Therefore, the composition obtained from the supernatant of a monocyte culture may be used for the same purposes as PRP.

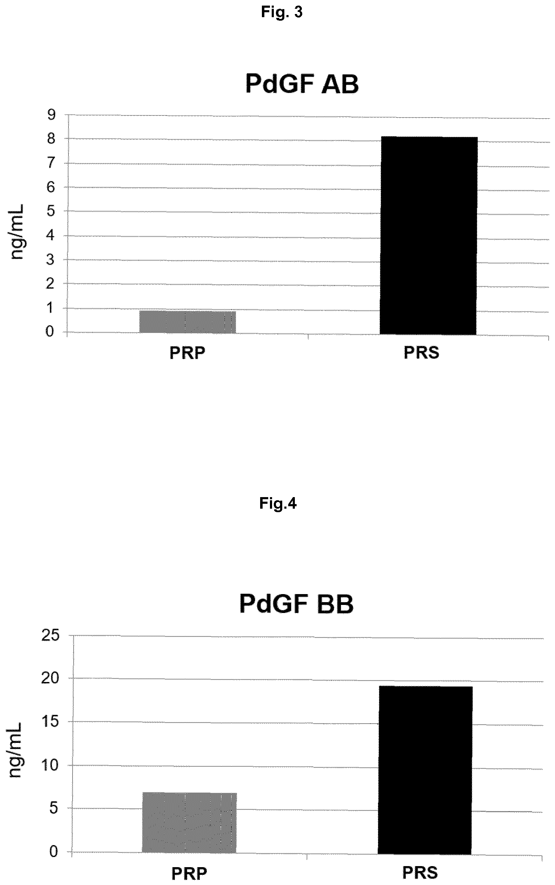

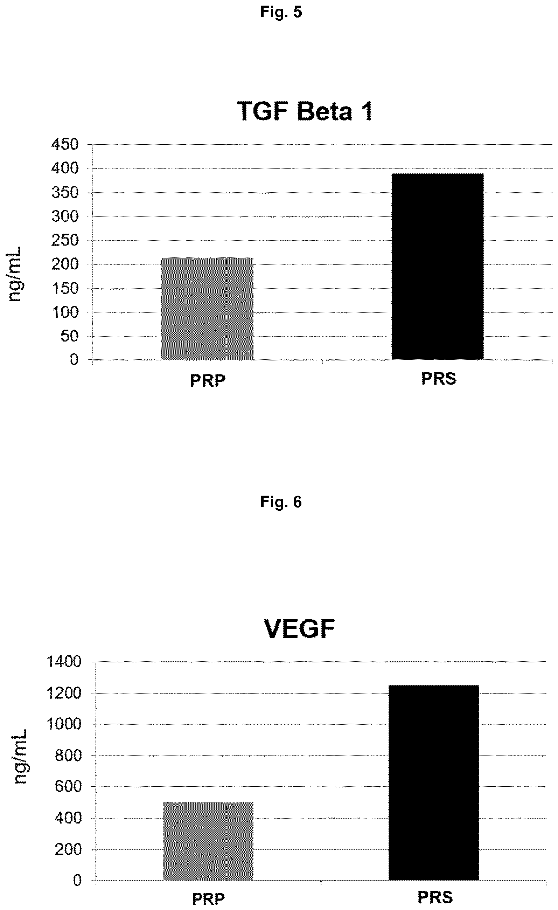

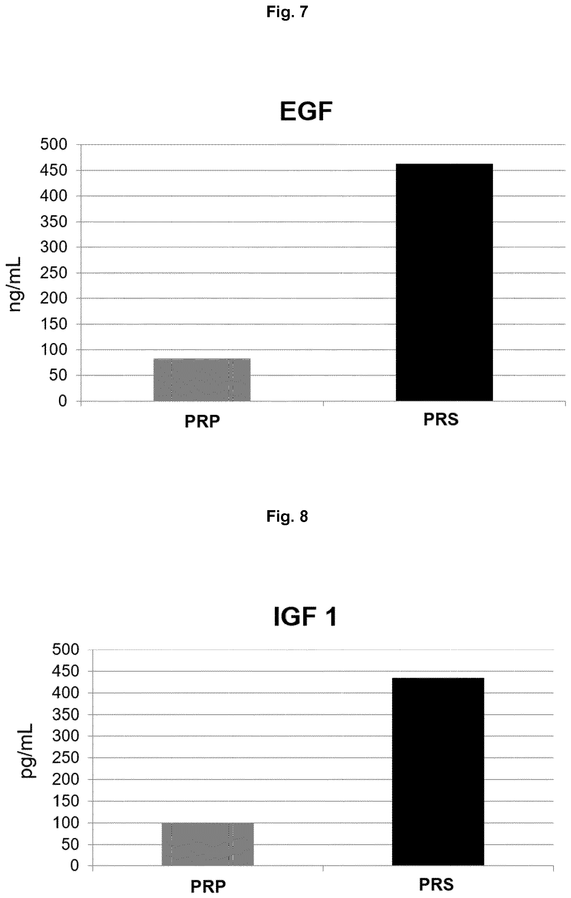

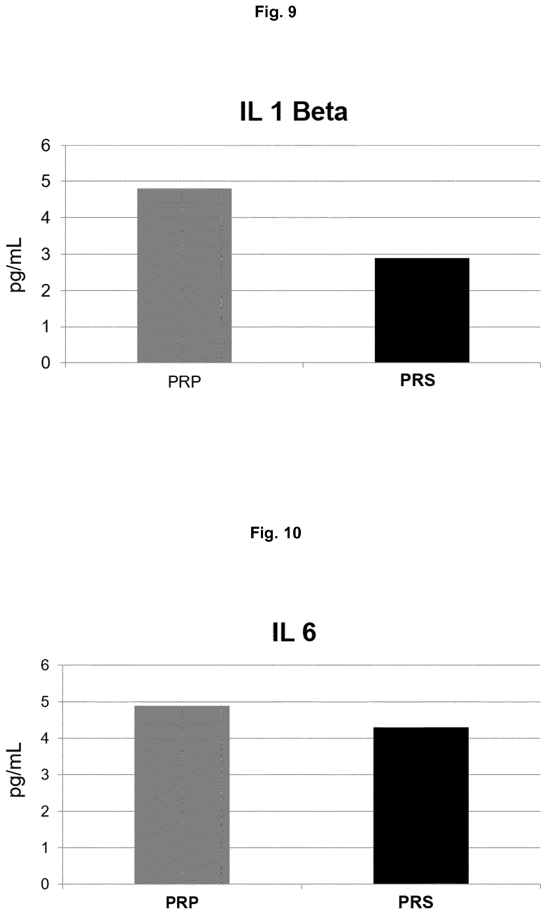

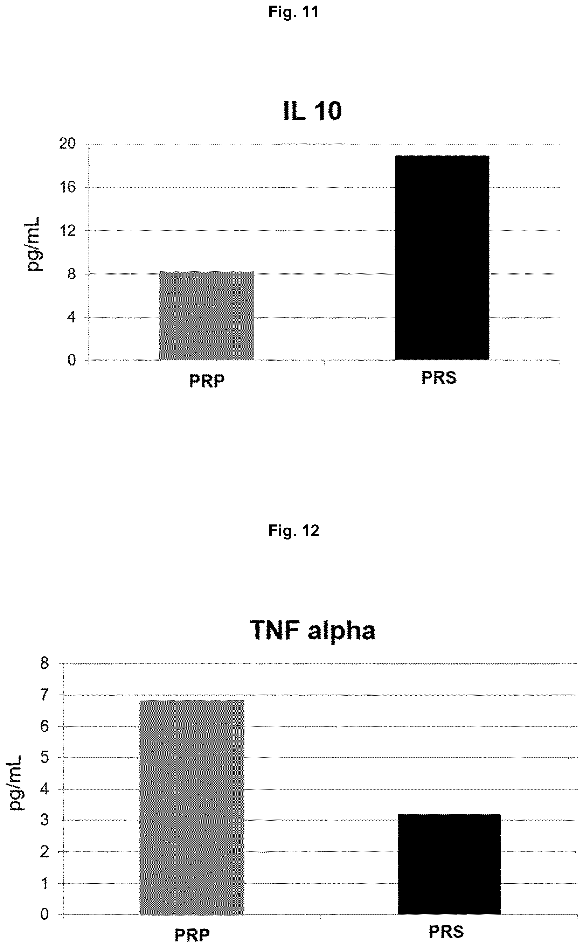

The cytokines and growth factors in the composition obtained from the example 1 (PRS) and the control sample (PRP) were quantified by multiplex analysis in a BioRad^{®} spectrophotometer. The compounds analyzed were: the platelet-derived growth factor AA (PdGF AA, Figure 2), the platelet-derived growth factor AB (PdGF AB, Figure 3), the platelet-derived growth factor BB (PdGF BB, Figure 4), the transforming growth factor beta (TGF beta, Figure 5), the vascular endothelial growth factor (VEGF, Figure 6), the epidermal growth factor (EGF, Figure 7), the insulin-like growth factor 1 (IGF 1, Figure 8), the interleukin 1 beta (IL 1 beta, Figure 9), the of interleukin 6 (IL 6, Figure 10), the interleukin 10 (IL 10, Figure 11) and the tumor necrosis factor alpha (TNF alpha, Figure 12).

As it may see from figures 2 to 8, the amount of all the growing factors PdGF AA, PdGF AB, PdGF BB, TGF beta 1, VEGF, EGF and IGF are significantly increased in the composition obtained from the supernatant of the culture, when compared with standard PRP.

The growth factors PDGF, TGF-β, IGF 1, VEGF and EGF are related to the cell migration and proliferation, playing an important role in the healing of muscular injuries. Moreover, IL 1 Beta and IL 6 are pro-inflammatory interleukins, whereas IL 10 is an antiinflammatory interleukin. The balance among them modulates the inhibitory or stimulatory signals during the inflammation process, in an autocrine, paracrine and endocrine form.

### Example 3: Clinical assays

The compositions obtained from the method of example 1 were tested in the treatment of injuries in muscles, tendons, ligaments, cartilages and bones in a total of 327 patients suffering from any of those injuries. Each tissue was treated with a composition obtained by culturing the monocytes with cells of the same type as the target tissue (tissue-specific compositions). The patients practiced sport activities such as football, tennis, running, athletics, trekking, paddle and basketball. The age of the subjects was between 15 and 58 years old (average age 36 years old). 37 % of the patients were male and 33 % were female subjects.

The injuries of each case were classified according to the Rutland scale of patellar tendinopathy, as indicated in Table 1 (Rutland M et al., 2010. N Am J Sports Phys Ther 5(3): 166).

**Table 1. Phases of patellar tendinopathy**

| **Phases** | **Blazina jumper's knee Scale** | **Kennedy tendinopathy stages** |
|---|---|---|
| **Phase 1** | Pain after activity only | Pain after activity |
| **Phase 2** | Pain/discomfort during and after activity with the subject still able to perform at a satisfactory level (does not interfere with participation) | Pain at the beginning and after activity |
| **Phase 3** | Pain during and after activity with more prolonged, with subject having progressively increasing difficulty in performing at a satisfactory level (interferes with competition) | Pain at the beginning, during, and after activity, but the performance is not affected |
| **Phase 4** | Complete tendon disruption | Pain at the beginning, during and after activity, and the performance is affected |

The subjects treated in the assay were classified as indicated in Table 2 according to the level of injury for each case:

**Table 2. Classification of the patients according to the type and severity of the injury.**

| **Injury** | **Cases (N)** | **Phase 2** | **Phase 3** | **Phase 4** |
|---|---|---|---|---|
| Patellar tendinosis | 22 | 12 | 10 | 0 |
| Achilles tendinitis | 19 | 8 | 11 | 0 |
| Muscle rupture IT | 28 | 14 | 14 | 0 |
| Muscle rupture RA | 19 | 10 | 9 | 0 |
| Gonarthrosis | 71 | 45 | 26 | 0 |
| Chondromalacia patellae | 66 | 34 | 32 | 0 |
| Anterior cruciate ligament rupture | 14 | 8 | 6 | 0 |
| Dynamic osteopathy of the pubis | 38 | 18 | 12 | 8 |
| astragalus fibular ligament sprain | 50 | 35 | 15 | 0 |

In order to assess the recovery in the subjects' tissue, the VISA score was used (Victoria Institute of Sport Assessment, Visentini PJ et al., 1998. J Sci Med Sport 1(1):22).

### Example 4: Treatment of patellar tendinosis

22 subjects suffering from patellar tendinosis (or tendinopathy) were selected. Patellar tendinosis is a musculoskeletal disorder affecting both recreational and elite athletes potentially leading to disability lasting several months. 12 of these subjects presented an initial phase II and 10, suffered an initial phase III. In order to assess the recovery in the subjects' tissue, the VISA score was used.

The composition was injected into the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

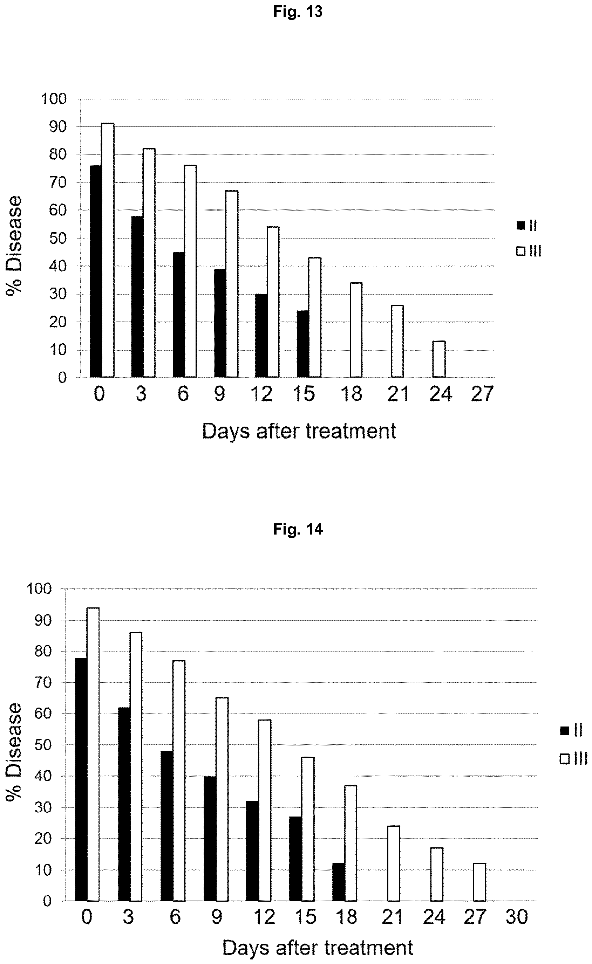

**Figure 13** shows the evolution of the percentage of scale of each phase of disease, from day 0 (control) until day 27.

Almost the 50 % of the patients with phase II injuries which were treated with the composition could return to the sport practice on day 3 after the treatment (PRS). On day 18, all these patients were performing sports. Moreover, the recovery time of the patients was even less than other treatments, such as PRS. After 27 days, all the patients with phase III injuries had returned to normal sport activity.

### Example 5: Treatment of Achilles tendinitis

19 subjects suffering from patellar tendinosis (or tendinopathy) were selected. Patellar tendinosis is a musculoskeletal disorder affecting both recreational and elite athletes potentially leading to disability lasting several months. 8 of these subjects presented an initial phase II and 11, suffered an initial phase III.

The composition was injected peri-tendon and intra-tendon in aseptic conditions in the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

**Figure 14** shows the evolution of the percentage of scale of each phase of disease, from day 0 (control) until day 30. On day 6, more than the 50 % of the patients with phase II injuries which were treated with the composition could return to the sport practice after the treatment (PRS).

### Example 6: Treatment of muscle tear

47 subjects suffering from muscle tear were selected. 28 of the subjects suffered hamstrings and 19 suffered a rupture of the quadriceps anterior rectum. 24 of the subjects presented an initial phase II and 23, suffered an initial phase III. In order to evaluate the effect of the composition in each subject, it was evaluated the percentage of players who got back to sports activity (RTP) after the treatment.

The composition was injected peri-tendon and intra-tendon in aseptic conditions in the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

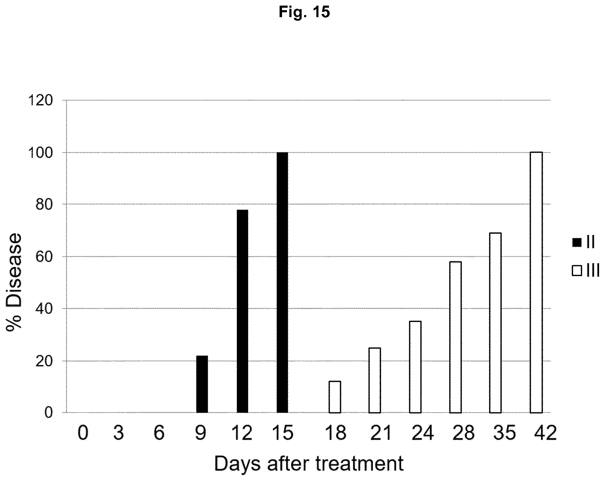

**Figure 15** shows the result of the treatment with the percentage of patients which returned to sport activity in a specific day. In grade II injuries, more than 50% of patients treated with PRS returned to sports on the 10th day after the treatment, 100% being on the 15th day after completion of the treatment.

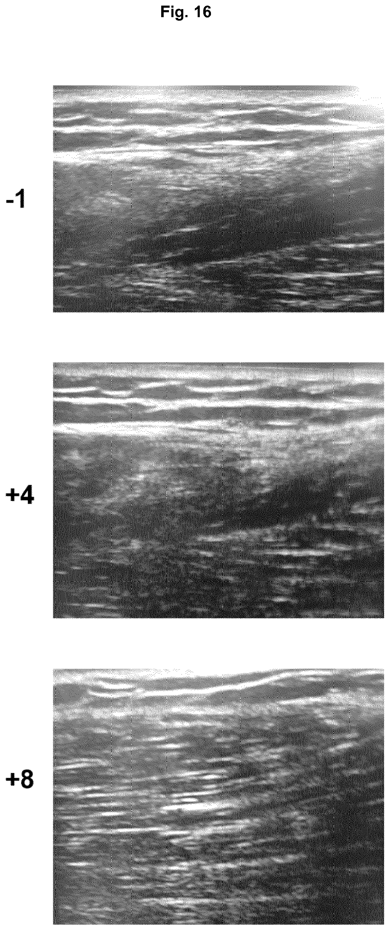

In order to evaluate the progression of the recovery of the injury, a follow-up of one of the patients, a 36 years old male with a fibrillar tear lower third of the biceps femoris, performed, in order to evaluate the healing of the muscle. **Figure 16** shows the result of an echography done on the day previous to the treatment (-1), the day 4 after the treatment (+4) and the day 8 after the treatment (+8).

From these results, it may be concluded that the patients to who the composition was applied, improved during the treatment, recovering from the injuries.

### Example 7: Treatment of gonarthrosis

71 subjects suffering from gonarthrosis were selected. 45 of the subjects presented an initial phase II and 26, suffered an initial phase III. In order to evaluate the effect of the composition in each subject, it was evaluated the functional and knee pain scale WOMAC scale (The Western Ontario and McMaster Universities Osteoarthritis Index).

The composition was injected intra-articular in aseptic conditions in the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

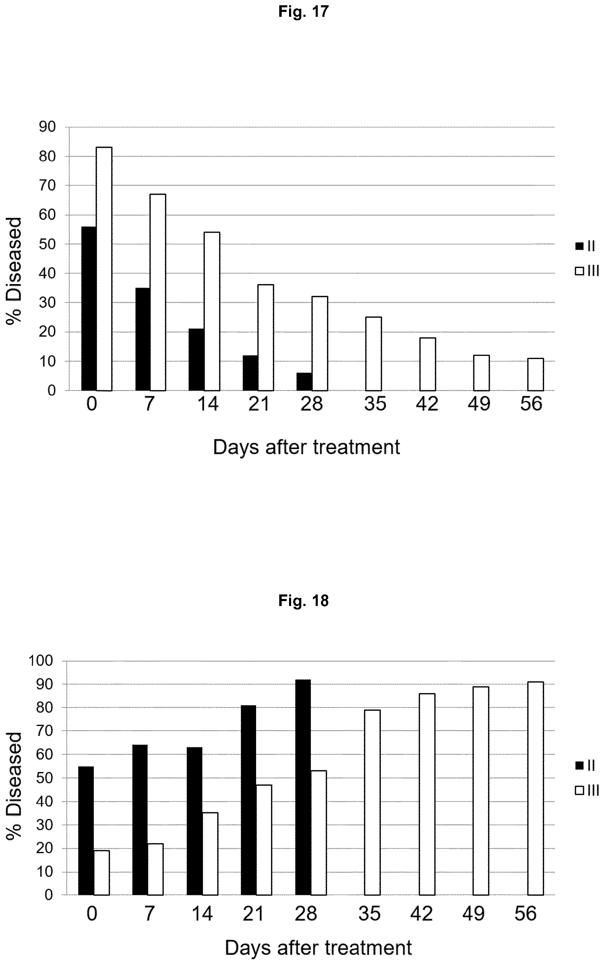

**Figure 17** shows the result of the treatment with the percentage of patients which returned to sport activity in a specific day. In grade II injuries, more than 60% of patients treated with PRS returned to sports on the 7th day after the treatment, 100% being on the 35th day after completion of the treatment.

From these results, it may be concluded that the patients to who the composition was applied, improved during the treatment, recovering from the injuries.

### Example 8: Treatment of chondromalacia patellae

66 subjects suffering from gonarthrosis were selected. 34 of the subjects presented an initial phase II and 32, suffered an initial phase III. In order to evaluate the effect of the composition in each subject, it was evaluated the international KOOS scale for the function and knee pain evaluation (Knee injury and Osteoarthritis Outcome Score.

The composition was injected intra-articular in aseptic conditions in the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

**Figure 18** shows the result of the treatment with the percentage of patients which returned to sport activity in a specific day (RTP, return to play).

### Example 9: Treatment of anterior cruciate ligament rupture (ACL)

14 subjects suffering from gonarthrosis were selected. 8 of the subjects presented an initial phase II and 6, suffered an initial phase III. In order to evaluate the effect of the composition in each subject, it was evaluated the KOOS scale. The phase III case were under semitendinosus plastic surgery.

The composition was injected intra-articular in aseptic conditions in the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

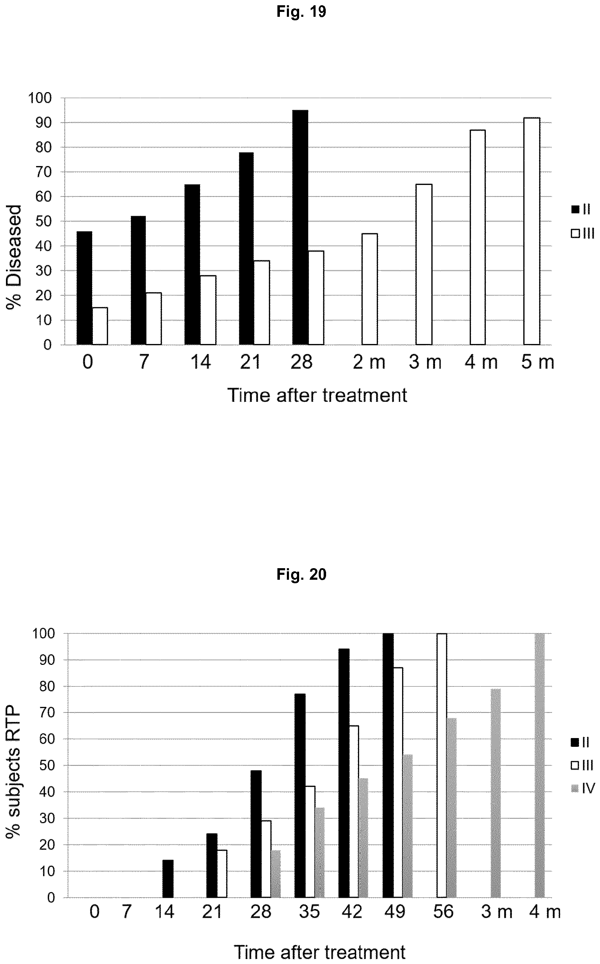

**Figure 19** shows the result of the treatment with the percentage of patients which returned to sport activity in a specific day.

### Example 10: Treatment of dynamic osteopathy of the pubis (DOC)

38 subjects suffering from DOC were selected. 18 of the subjects presented an initial phase II, 12, suffered an initial phase III and 8 an initial phase IV. In order to evaluate the effect of the composition in each subject, it was evaluated the percentage of players who got back to sports activity (RTP) after the treatment.

The composition was injected intra-articular in aseptic conditions in the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

**Figure 20** shows the result of the treatment with the percentage of patients which returned to sport activity in a specific day.

### Example 11: Treatment of astragalus fibular ligament sprain (AFL)

50 subjects suffering from LPA were selected. 35 of the subjects presented an initial phase II and 15, suffered an initial phase III. In order to evaluate the effect of the composition in each subject, it was evaluated the FAAM scale (Foot and Ankle Ability Measure.

The composition was injected intra-articular in aseptic conditions in the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

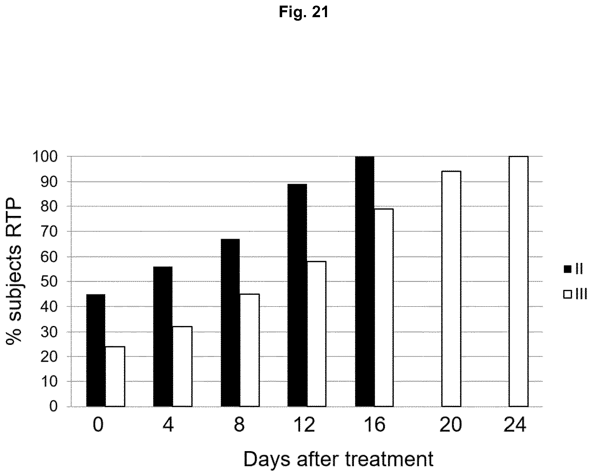

**Figure 21** shows the result of the treatment with the percentage of patients which returned to sport activity in a specific day.

## Claims

1. Method for obtaining a composition, comprising the following steps:
a) purification of monocytes from a sample in sterile conditions;
b) culturing the purified monocytes in serum free medium with tissue-specific cells;
c) collection the supernatant comprising growth factors and/or cytokines produced by the culture of step b);
d) cryopreservation of the supernatant collected in step c) at a temperature between -35 and -165 °C,
wherein the tissue-specific cells are selected from the group consisting of myocytes, skeletal muscle cells, muscle satellite cells, tenocytes, Schwann cells, chondrocytes, synoviocytes, lymphocytes, hair follicle cells, myocardia cells, mesenchymal stem cells or combinations thereof;
wherein the composition is for tissue regeneration of musculoskeletal injuries.

2. The method of claim 1, additionally comprising the step of:
e) reconstitution of the growth factors cryopreserved by maintaining the composition between 25 and 30 °C between for 10 and 60 minutes.

3. The method of claims 1 or 2, wherein the tissue-specific cells are obtained from a non-injured tissue.

4. The method of claims 1 or 2, wherein the tissue-specific cells are obtained from an injured tissue.

5. The method of claims 1 to 4, wherein the culture step b) further comprises adult mesenchymal cells.

6. The method of claims 1 to 5, wherein the sample in step a) is from the same donor as the tissue-specific cells.

7. Composition for tissue regeneration obtained by a method consisting of the following steps:
a) purification of monocytes from a sample in sterile conditions;
b) culturing the purified monocytes in serum free medium with tissue-specific cells;
c) optionally, adding a macrophage colony-stimulating factor;
d) collection the supernatant comprising growth factors and/or cytokines produced by the culture of step b);
e) cryopreservation of the supernatant collected in step c) at a temperature between -35 and -165 °C,
wherein the tissue-specific cells are selected from the group consisting of myocytes, skeletal muscle cells, muscle satellite cells, tenocytes, Schwann cells, chondrocytes, synoviocytes, lymphocytes, hair follicle cells, myocardia cells, mesenchymal stem cells or combinations thereof.

8. The composition according to claim 7, comprising platelets, red cells or leukocytes in an amount of less than 0.001% of the total amount of components in the sample.

9. Pharmaceutical composition comprising the composition of claims 7 or 8 and a pharmaceutical acceptable carrier.

10. A composition obtainable by a method consisting of the following steps:
a) purification monocytes from a sample;
b) culturing the purified monocytes in serum free medium with tissue-specific cells selected from the group consisting of human chondrocytes, human synoviocytes, human Schwann cells, human skeletal muscle cells, human muscle satellite cells, human dermal fibroblasts, Human tenocytes, human mesenchymal stem cells; and
c) collection the supernatant comprising growth factors and/or cytokines produced in the culture of step b);
for use in a method of treatment a musculoskeletal disease selected from tendinosis, muscle fibrillary break, tendinitis, bursitis, synovitis, arthrosis, cartilage break or chondropathies
wherein the composition is administered in a tissue comprising cells of the same type as the tissue-specific cells of step b).

11. The composition for use according to claim 10, wherein the composition is administered to a subject different from the subject donor of the monocytes or the tissue-specific cells.

12. A composition obtainable by a method consisting of the following steps:
a) purification monocytes from a sample in sterile conditions;
b) culturing the purified monocytes in serum free medium with tissue-specific cells selected from the group consisting of human chondrocytes, human synoviocytes, human Schwann cells, human skeletal muscle cells, human muscle satellite cells, human dermal fibroblasts, Human tenocytes, human mesenchymal stem cells;
c) collection the supernatant comprising growth factors and/or cytokines produced in the culture of step b);
d) cryopreservation of the supernatant collected in step c) at a temperature between -35 and -165 °C; and
e) reconstitution of the supernatant of step d) by maintaining it between 25 and 30 °C,
for use in a method of treatment a disease selected from tendinosis, muscle fibrillary break, tendinitis, bursitis, synovitis, arthrosis, cartilage break or chondropathies
wherein step e) is performed for 10 to 60 minutes prior to its use, and
wherein the composition is administered in a tissue comprising cells of the same type as the tissue-specific cells of step b).

## Patentansprüche

1. Verfahren zum Erhalten einer Zusammensetzung, welches folgende Schritte umfasst:
a) Aufreinigen von Monozyten aus einer Probe unter sterilen Bedingungen;
b) Kultivieren der aufgereinigten Monozyten in serumfreiem Medium mit gewebespezifischen Zellen;
c) Sammeln des Überstands, umfassend Wachstumsfaktoren und/oder Zytokine, hergestellt durch die Kultivierung von Schritt b);
d) Kryokonservieren des in Schritt c) gesammelten Überstands bei einer Temperatur zwischen -35 und -165 °C,
wobei die gewebespezifischen Zellen ausgewählt sind aus der Gruppe bestehend aus Myozyten, Skelettmuskelzellen, Satellitenzellen des Muskels, Tenozyten, Schwann-Zellen, Chondrozyten, Synoviozyten, Lymphozyten, Haarfollikelzellen, Myokardzellen, mesenchymalen Stammzellen oder Kombinationen davon;
wobei die Zusammensetzung für die Geweberegeneration von muskuloskelettalen Verletzungen bestimmt ist.

2. Verfahren nach Anspruch 1, welches zusätzlich die folgenden Schritte umfasst:
e) Rekonstituieren der kryokonservierten Wachstumsfaktoren durch Halten der Zusammensetzung zwischen 25 und 30 °C zwischen 10 und 60 Minuten.

3. Verfahren nach Anspruch 1 oder 2, wobei die gewebespezifischen Zellen aus einem nicht verletzten Gewebe erhalten werden.

4. Verfahren nach Anspruch 1 oder 2, wobei die gewebespezifischen Zellen aus einem verletzten Gewebe erhalten werden.

5. Verfahren nach Anspruch 1 bis 4, wobei der Kultivierungsschritt b) ferner adulte mesenchymale Zellen umfasst.

6. Verfahren nach Anspruch 1 bis 5, wobei die Probe in Schritt a) von dem gleichen Spender stammt wie die gewebespezifischen Zellen.

7. Zusammensetzung zur Geweberegeneration, erhalten durch ein Verfahren, welches aus den folgenden Schritten besteht:
a) Aufreinigen von Monozyten aus einer Probe unter sterilen Bedingungen;
b) Kultivieren der aufgereinigten Monozyten in serumfreiem Medium mit gewebespezifischen Zellen;
c) gegebenenfalls Hinzufügen eines Makrophagen-Kolonie-stimulierenden Faktors;
d) Sammeln des Überstands, umfassend Wachstumsfaktoren und/oder Zytokine, hergestellt durch die Kultivierung von Schritt b);
e) Kryokonservieren des in Schritt c) gesammelten Überstands bei einer Temperatur zwischen -35 und -165 °C,
wobei die gewebespezifischen Zellen ausgewählt sind aus der Gruppe bestehend aus Myozyten, Skelettmuskelzellen, Satellitenzellen des Muskels, Tenozyten, Schwann-Zellen, Chondrozyten, Synoviozyten, Lymphozyten, Haarfollikelzellen, Myokardzellen, mesenchymalen Stammzellen oder Kombinationen davon.

8. Zusammensetzung nach Anspruch 7, umfassend Blutplättchen, rote Blutkörperchen oder Leukozyten in einer Menge von weniger als 0,001 % der Gesamtmenge der Komponenten in der Probe.

9. Pharmazeutische Zusammensetzung, umfassend die Zusammensetzung nach Anspruch 7 oder 8 und einen pharmazeutisch annehmbaren Träger.

10. Zusammensetzung, erhältlich durch ein Verfahren, welches aus den folgenden Schritten besteht:
a) Aufreinigen von Monozyten aus einer Probe;
b) Kultivieren der aufgereinigten Monozyten in serumfreiem Medium mit gewebespezifischen Zellen, ausgewählt aus der Gruppe bestehend aus humanen Chondrozyten, humanen Synoviozyten, humanen Schwann-Zellen, humanen Skelettmuskelzellen, humanen Satellitenzellen des Muskels, humanen dermalen Fibroblasten, humanen Tenozyten, humanen mesenchymalen Stammzellen; und
c) Sammeln des Überstands, umfassend Wachstumsfaktoren und/oder Zytokine, hergestellt in der Kultivierung von Schritt b);
zur Verwendung in einem Verfahren zur Behandlung einer muskuloskelettalen Krankheit, ausgewählt aus Tendinose, Muskelfaserriss, Tendinitis, Bursitis, Synovitis, Arthrose, Knorpelbruch oder Chondropathien
wobei die Zusammensetzung in einem Gewebe verabreicht wird, umfassend Zellen des gleichen Typs wie die gewebespezifischen Zellen von Schritt b).

11. Zusammensetzung zur Verwendung nach Anspruch 10, wobei die Zusammensetzung einem Individuum verabreicht wird, das sich von dem Individuum, das die Monozyten oder die gewebespezifischen Zellen spendet, unterscheidet.

12. Zusammensetzung, erhältlich durch ein Verfahren, welches aus den folgenden Schritten besteht:
a) Aufreinigen von Monozyten aus einer Probe unter sterilen Bedingungen;
b) Kultivieren der aufgereinigten Monozyten in serumfreiem Medium mit gewebespezifischen Zellen, ausgewählt aus der Gruppe bestehend aus humanen Chondrozyten, humanen Synoviozyten, humanen Schwann-Zellen, humanen Skelettmuskelzellen, humanen Satellitenzellen des Muskels, humanen dermalen Fibroblasten, humanen Tenozyten, humanen mesenchymalen Stammzellen;
c) Sammeln des Überstands, umfassend Wachstumsfaktoren und/oder Zytokine, hergestellt in der Kultivierung von Schritt b);
d) Kryokonservieren des in Schritt c) gesammelten Überstands bei einer Temperatur zwischen -35 und -165 °C; und
e) Rekonstituieren des Überstands von Schritt d), indem er zwischen 25 und 30 °C gehalten wird,
zur Verwendung in einem Verfahren zur Behandlung einer Krankheit, ausgewählt aus Tendinose, Muskelfaserriss, Tendinitis, Bursitis, Synovitis, Arthrose, Knorpelbruch oder Chondropathien
wobei Schritt e) vor der Verwendung 10 bis 60 Minuten lang durchgeführt wird und
wobei die Zusammensetzung in einem Gewebe verabreicht wird, umfassend Zellen des gleichen Typs wie die gewebespezifischen Zellen von Schritt b).

## Revendications

1. Procédé pour obtenir une composition, comprenant les étapes suivantes :
a) purification de monocytes à partir d'un échantillon dans des conditions stériles ;
b) culture des monocytes purifiés dans du milieu exempt de sérum avec des cellules spécifiques à des tissus ;
c) collecte du surnageant comprenant des facteurs de croissance et/ou des cytokines produites par la culture de l'étape b) ;
d) cryoconservation du surnageant collecté dans l'étape c) à une température comprise entre -35 et -165°C,
dans lequel les cellules spécifiques à des tissus sont choisies parmi le groupe constitué des myocytes, des cellules du muscle squelettique, des cellules satellites musculaires, des ténocytes, des cellules de Schwann, des chondrocytes, des synoviocytes, des lymphocytes, des cellules du follicule pileux, des cellules myocardiaques, des cellules souches mésenchymateuses ou des combinaisons de celles-ci ;
dans lequel la composition est pour la régénération tissulaire de lésions musculo-squelettiques.

2. Procédé selon la revendication 1, comprenant en outre l'étape de :
e) reconstitution des facteurs de croissance crioconservés en maintenant la composition entre 25 et 30°C pendant entre 10 et 60 minutes.

3. Procédé selon les revendications 1 ou 2, dans lequel les cellules spécifiques à des tissus sont obtenues à partir d'un tissu non-lésé.

4. Procédé selon les revendications 1 ou 2, dans lequel les cellules spécifiques à des tissus sont obtenues à partir d'un tissu lésé.

5. Procédé selon les revendications 1 à 4, dans lequel l'étape de culture b) comprend en outre des cellules mésenchymateuses d'adulte.

6. Procédé selon les revendications 1 à 5, dans lequel l'échantillon dans l'étape a) est du même donneur que les cellules spécifiques à des tissus.

7. Composition pour la régénération tissulaire obtenue par le biais d'un procédé consistant en les étapes suivantes :
a) purification de monocytes à partir d'un échantillon dans des conditions stériles ;
b) culture des monocytes purifiés dans du milieu exempt de sérum avec des cellules spécifiques à des tissus ;
c) optionnellement, ajout d'un facteur de stimulation des colonies de macrophages ;
d) collecte du surnageant comprenant des facteurs de croissance et/ou des cytokines produites par la culture de l'étape b) ;
e) cryoconservation du surnageant collecté dans l'étape c) à une température comprise entre -35 et -165°C,
dans laquelle les cellules spécifiques à des tissus sont choisies parmi le groupe constitué des myocytes, des cellules du muscle squelettique, des cellules satellites musculaires, des ténocytes, des cellules de Schwann, des chondrocytes, des synoviocytes, des lymphocytes, des cellules du follicule pileux, des cellules myocardiaques, des cellules souches mésenchymateuses ou des combinaisons de celles-ci.

8. Composition selon la revendication 7, comprenant des plaquettes, des globules rouges ou des leucocytes dans une quantité de moins de 0,001% de la quantité totale des composants dans l'échantillon.

9. Composition pharmaceutique comprenant la composition selon les revendications 7 ou 8 et un véhicule pharmaceutique acceptable.

10. Composition pouvant être obtenue par le biais d'un procédé consistant en les étapes suivantes :
a) purification de monocytes à partir d'un échantillon ;
b) culture des monocytes purifiés dans du milieu exempt de sérum avec des cellules spécifiques à des tissus choisies parmi le groupe constitué des chondrocytes humains, des synoviocytes humains, des cellules de Schwann humaines, des cellules du muscle squelettique humaines, des cellules satellites musculaires humaines, des fibroblastes dermiques humains, des ténocytes humains, des cellules souches mésenchymateuses humaines ; et
c) collecte du surnageant comprenant des facteurs de croissance et/ou des cytokines produites dans la culture de l'étape b) ;
pour son utilisation dans un procédé de traitement d'une maladie musculo-squelettique choisie parmi la tendinose, la rupture des fibrilles musculaires, la tendinite, la bursite, la synovite, l'arthrose, la rupture du cartilage ou les chondryopathies
dans laquelle la composition est administrée dans un tissu comprenant des cellules du même type que les cellules spécifiques à des tissus de l'étape b).

11. Composition pour son utilisation selon la revendication 10, dans laquelle la composition est administrée à un sujet différent du sujet donneur des monocytes ou des cellules spécifiques à des tissus.

12. Composition pouvant être obtenue par un procédé consistant en les étapes suivantes :
a) purification de monocytes à partir d'un échantillon dans des conditions stériles ;
b) culture des monocytes purifiés dans du milieu exempt de sérum avec des cellules spécifiques à des tissus choisies parmi le groupe constitué des chondrocytes humains, des synoviocytes humains, des cellules de Schwann humaines, des cellules du muscle squelettique humaines, des cellules satellites musculaires humaines, des fibroblastes dermiques humains, des ténocytes humains, des cellules souches mésenchymateuses humaines ;
c) collecte du surnageant comprenant des facteurs de croissance et/ou des cytokines produites dans la culture de l'étape b) ;
d) cryoconservation du surnageant collecté dans l'étape c) à une température comprise entre -35 et -165°C ; et
e) reconstitution du surnageant de l'étape d) en le maintenant entre 25 et 30°C,
pour son utilisation dans un procédé de traitement d'une maladie choisie parmi la tendinose, la rupture des fibrilles musculaires, la tendinite, la bursite, la synovite, l'arthrose, la rupture du cartilage ou les chondryopathies
dans laquelle étape e) est réalisée pendant 10 à 60 minutes avant son utilisation, et
dans laquelle la composition est administrée dans un tissu comprenant des cellules du même type que les cellules spécifiques à des tissus de l'étape b).
